# EUROPEAN PATENT SPECIFICATION

(11) **EP 0 710 097 B1**
(45) Date of publication and mention of the grant of the patent: **28.01.1998**
(21) Application number: 94922674.0
(22) Date of filing: 22.07.1994
(51) Int. Cl.: A61F 13/15

(54) **ABSORBENT ARTICLES HAVING FIXED LENGTH UNDERGARMENT COVERING COMPONENTS THAT AUTOMATICALLY WRAP THE SIDES OF UNDERGARMENTS**
SAUGFÄHIGE ARTIKEL VORGESCHRIEBENER LÄNGE MIT BESTANDTEILEN DIE AUTOMATISCH DIE SEITEN DER UNTERWÄSCHE UMWICKELN
ARTICLES ABSORBANTS POSSEDANT DES ELEMENTS DE COUVERTURE DES SOUS-VETEMENTS A LONGUEUR FIXE ENVELOPPANT AUTOMATIQUEMENT LES COTES DES SOUS-VETEMENTS

(30) Priority: 22.07.1993 US 96121; 20.09.1993 US 124180
(43) Date of publication of application: 08.05.1996
(73) Proprietor: THE PROCTER & GAMBLE COMPANY, Cincinnati, Ohio 45202 (US)
(72) Inventor: WEINBERGER, Eric, Patton, Hamilton, OH 45011 (US); HAMMONS, John, Lee, Hamilton, OH 45011 (US); LAMPSON, Patricia, Lee, Cincinnati, OH 45205 (US); LINMAN, E., Kelly, Cincinnati, OH 45245 (US); BAMBER, Jeffrey, Vincent, Cincinnati, OH 45244 (US)
(74) Representative: Hirsch, Uwe Thomas
(86) International application number: US9408255
(87) International publication number: WO9503023

(56) References cited:
- EP-A- 0 235 854
- EP-A- 0 331 018
- EP-A- 0 337 438
- FR-A- 2 437 203
- US-A- 5 181 563

## Description

### FIELD OF THE INVENTION

The present invention relates to absorbent articles such as sanitary napkins, panty liners, and incontinence pads. More particularly, the present invention relates to sanitary napkins that have fixed length undergarment covering components (or "side wrapping elements") that automatically wrap the sides of a wearer's undergarments when the undergarments are pulled up which provide a alternative to conventional side flaps.

### BACKGROUND OF THE INVENTION

Absorbent articles such as sanitary napkins, pantiliners, and incontinence pads are devices that are typically worn in the crotch region of a undergarment. These devices are designed to absorb and retain liquid and other discharges from the human body and to prevent body and clothing soiling. Sanitary napkins are a type of absorbent article worn by women in a pair of panties that is normally positioned between the wearer's legs, adjacent to the perineal area of the body. Sanitary napkins both with and without side flaps (or wings) are disclosed in the literature and are available in the marketplace.

Generally when sanitary napkins are provided with flaps, the flaps extend laterally from a central absorbent means and are intended to be folded around the edges of the wearer's panties in the crotch region. Commonly, the flaps are provided with an attachment means for either affixing the flaps to the underside of the wearer's panties or to the opposing flap. The flaps are particularly effective for preventing exudates from soiling the edges of the wearer's panties.

Sanitary napkins having flaps of various types are disclosed in U.S. Patent 4,687,478, entitled "Shaped Sanitary Napkin With Flaps", which issued to Van Tilburg on August 18, 1987; U.S. Patent 4,608,047, entitled "Sanitary Napkin Attachment Means", which issued to Mattingly on August 26, 1986; U.S. Patent 4,589,876, entitled "Sanitary Napkin", which issued to Van Tilburg on May 20, 1986 and its Reexamination Patent No. B1 4,589,876, Certificate of Reexamination issued April 27, 1993; U.S. Patent 4,285,343, entitled "Sanitary Napkin", which issued to McNair on August 25, 1981; U.S. Patent 3,397,697, entitled "Disposable Sanitary Shield For Undergarments", which issued to Rickard on August 20, 1968; and, U.S. Patent 2,787,271, entitled "Sanitary Napkin", which issued to Clark on April 2, 1957.

While sanitary napkins having flaps are commonly viewed as providing better protection against soiling as compared to sanitary napkins without flaps, some women find applying sanitary napkins having flaps to be inconvenient for various reasons. For instance, some women find it to be difficult to attach the flaps to the underside of the crotch of their panties. This can be due to factors such as the tendency for the adhesive fasteners on the flaps to stick to themselves or to other parts of the sanitary napkin. As a result, some women still prefer a sanitary napkin without flaps. In addition, some women who generally prefer a sanitary napkin with flaps, occasionally prefer a sanitary napkin without flaps. Therefore, there is a need for a sanitary napkin which provides an alternative to sanitary napkins having conventional side flaps while still providing the protection of side flaps.

US-A-4,940,426 which is equivalent to
EP-A-331 018 discloses sanitary napkins having side flaps which are tensioned in longitudinal direction typically by the addition of an elastic material without providing automatical wrapping.
EP-A-337 438 discloses a sanitary napkin having biased hinges to connect side flaps. The flaps have to be unfolded prior to application of the napkin.

Thus, a need exists for an absorbent article, such as a sanitary napkin, that is

Several variations of sanitary napkins having conventional flaps that attempt to solve some, but not all of these problems are disclosed in the patent literature. For example, U.S. Patent 4,911,701 issued to Mavinkurve discloses a sanitary napkin having elastic strands for providing a greater convex shape to the body-facing portion of the central absorbent and for enabling adhesive-free placement of the flaps of a winged napkin embodiment into a pair of panties. The sanitary napkin described in the Mavinkurve patent, however, still appears to require the user to manipulate the flaps (by first flipping the flaps upward and then placing the flaps in her panties and flipping the flaps back down) since the flaps appear to be pre-disposed to be in a downward folded condition. The Mavinkurve patent also requires that individual elastic strands be attached in a contracted condition to the central absorbent portion of the napkin and/or to its wings or flaps. The napkins described in the Mavinkurve patent can, therefore, be difficult and expensive to manufacture. U.S. Patent 4,940,462 issued to Salerno discloses a sanitary napkin with longitudinally expandable flaps. The flaps are designed to fold over the exterior of the wearer's panty and then to longitudinally expand to conform with the contour of the panties. The Salerno patent, however, appears to require conventional adhesive fasteners to retain the flaps in place on the underside of the wearer's panties.

In particular, a need exists for a sanitary napkin having an alternative to conventional flaps which provides the protection from soiling of conventional flaps and which can conveniently and efficiently solve the problems caused when attempting to attach conventional flaps to the underside of the wearer's panties.

It is, therefore, an object of the present invention to provide an absorbent article, such as a sanitary napkin, that is able to provide coverage to the wearer's panties to reduce side soiling (i.e., staining of the edges of the panty crotch) without the use of conventional flaps.

It is another object of the present invention to provide an absorbent article, such as a sanitary napkin that automatically wraps around the sides of the wearer's panties by the simple action of the wearer pulling up her panties.

It is still another object of the present invention to provide an absorbent article, such as a sanitary napkin, that is able to wrap around the sides of the wearer's panties and stay without providing flaps having panty fasteners thereon, and without attaching separate elastic strands to the sanitary napkin.

These and other objects of the present invention will be more readily apparent when considered in reference to the following description and when taken in conjunction with the accompanying drawings.

### SUMMARY OF THE INVENTION

The present invention provides an absorbent article, such as a sanitary napkin. The sanitary napkin of the present invention has undergarment covering components (or "side wrapping elements") that automatically wrap the sides of a wearer's undergarments when the undergarments are pulled up. The sanitary napkin of the present invention provides coverage to the wearer's panties to reduce side soiling (i.e., staining of the edges of the panty crotch) without the use of conventional flaps which must be manually positioned into their in-use condition.

The sanitary napkin has a longitudinal dimension extending in a longitudinal direction and a transverse dimension extending in a transverse direction. The sanitary napkin comprises a main body portion and a pair of side wrapping elements. The main body portion comprises a liquid pervious topsheet, a liquid impervious backsheet joined to the topsheet, and an absorbent core positioned between the topsheet and the backsheet. The side wrapping elements comprise a pair of flexible elements that are joined to the main body portion and extend laterally outward beyond the longitudinal side edges of the main body portion to their distal edges. The side wrapping elements are pre-disposed to fold around the edges of the crotch region of the wearer's undergarments. The side wrapping elements have a fixed length distal edge that provides the side wrapping elements with a hoop-like structure that resists extension and that facilitates automatic flipping or wrapping of the side wrapping elements around the edges of the wearer's undergarment when the undergarment is pulled into its in-use condition. This automatic flipping action is facilitated when the main body portion of the sanitary napkin is placed in a wearer's panties and the main body portion assumes an upwardly arched configuration along its longitudinal centerline when the panties are pulled into contact with the wearer's body.

In one preferred embodiment, the side wrapping elements comprise a first region and a second region that have different flexibilities. In this embodiment, the first region preferably comprises a plurality of spaced apart softened, flexible portions of the side wrapping elements that are preferably extensible primarily in the transverse direction and are disposed laterally inward of the second region. The second region is less soft, less flexible, and preferably less extensible than the first region and is disposed along at least a portion of the distal edges of the side wrapping elements. The second region of the side wrapping elements maintains a line of tension along the distal edges of the side wrapping element that facilitates the automatic flipping of the side wrapping element around the side edges of the wearer's undergarment when the undergarment is pulled into its in-use condition.

In a second preferred embodiment, the side wrapping elements comprise a first region that is extensible primarily in the transverse direction and a less extensible second region that is disposed between portions of the first region. The side wrapping elements are free of elastic strands. The side wrapping elements in this second preferred embodiment can comprise a web material having a network of strainable regions formed therein. The less extensible second region maintains the side wrapping elements in tension when said first region is extended laterally outward to facilitate automatic flipping of the side wrapping elements around the side edges of the wearer's undergarment as the undergarment is raised into position.

In a third preferred embodiment, the side wrapping elements comprise a plurality of spaced apart regions or components that are gathered longitudinally inward along the distal edges of the side wrapping elements and joined together. The longitudinal gathering inward of the regions or components maintains a line of tension along the distal edges of the side wrapping element that facilitates automatic flipping of the side wrapping element around the side edges of the wearer's undergarment when the undergarment is pulled into its in-use condition.

The sanitary napkin of the present invention provides an alternative to sanitary napkins having conventional side flaps for several reasons. The side wrapping elements do not extend far enough outward beyond the side edges of the wearer's panties to cause any inconvenience to the wearer. The sanitary napkin requires no action on the part of the wearer in order to fold the side wrapping elements under their panties or to attach the same to their panties. The side wrapping elements, therefore, stay in place well enough to cover the sides edges of the wearer's panties without affixing them underneath the wearer's panties.

### BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings in which:
FIG. 1 is a perspective view of the garment-facing side of one embodiment of the sanitary napkin of the present invention with the side wrapping elements folded over the release paper on the garment-facing side.
FIG. 2 is another perspective view of the sanitary napkin shown in FIG. 1 showing the removal of the release paper and the unfolding of the side wrapping elements.
FIG. 3 is a perspective view showing the side wrapping elements of the sanitary napkin shown in FIG. 1 in their unfolded condition.
FIG. 4 is a cross-sectional view of the sanitary napkin shown in FIG. 3 taken along line 4-4 of FIG. 3.
FIG. 5 is a perspective view of the sanitary napkin shown in the preceding figures, taken from the body-facing side showing the folding of the side wrapping elements in use.
FIG. 5A is a schematic cross-sectional view of the three positions the side wrapping elements are preferably capable of assuming during wear.
FIG. 6 is a plan view of a second embodiment of the sanitary napkin of the present invention in which the side wrapping elements comprise a web material having a network of strainable distinct regions formed therein and reinforced by a scrim.
FIG. 7 is an end view of the sanitary napkin shown in FIG. 6.
FIG. 7A is a plan view of a polymeric web material having a strainable network which is used in the side wrapping elements of the sanitary napkin shown in FIGS. 6 and 7 (shown with the deformations facing toward the viewer).
FIGS. 7B-D are enlarged segmented perspective illustrations of the polymeric web material used in the side wrapping elements of the sanitary napkin shown in FIGS. 6 and 7 moving from an untensioned condition to progressively greater tensioned conditions.
FIG. 8 is a schematic plan view of a portion of an alternative version of the embodiment shown in FIGS. 6 and 7 in which the distal edge of the side wrapping element does not have the strainable network formed therein.
FIGS. 9 and 10 are perspective views of a third embodiment of a sanitary napkin of the present invention in which FIG. 9 shows the formation of side wrapping elements comprising a plurality of overlapping components that are gathered together and sealed with a seam and FIG. 10 shows the completely-formed product.

### DETAILED DESCRIPTION OF THE INVENTION

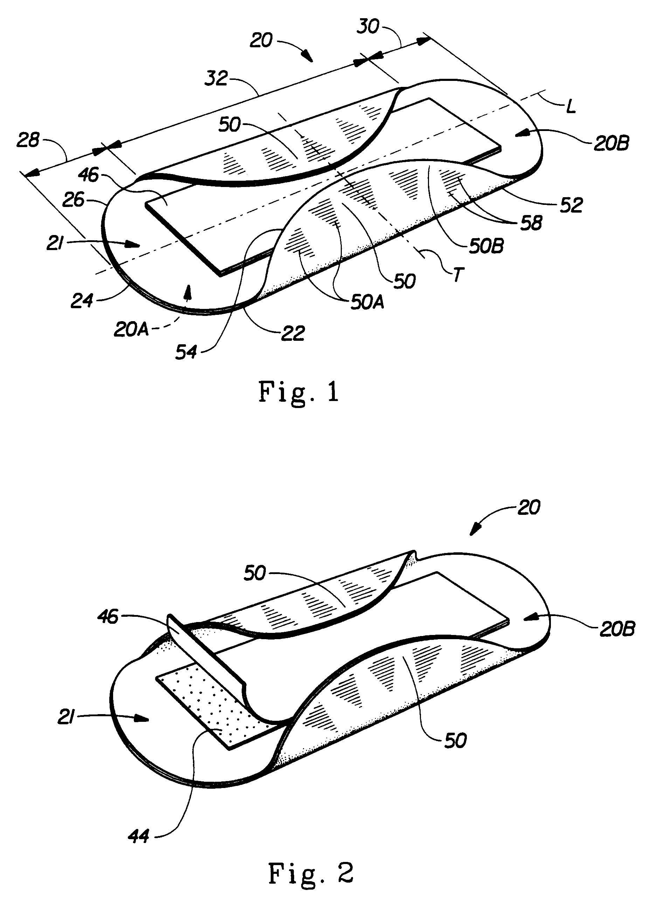

FIGS. 1-5 show one preferred embodiment of a disposable absorbent article of the present invention, sanitary napkin 20. The present invention relates to absorbent articles such as sanitary napkins that have a main body portion 21 and undergarment covering components (or "side wrapping elements") 50 that automatically wrap the sides of the wearer's panties when the wearer places the sanitary napkin in her panties and pulls her panties up.

The sanitary napkin 20 has two surfaces, a liquid pervious body-contacting surface or "body surface" 20A that is intended to be worn adjacent to the body of the wearer and a liquid impervious garment surface 20B. The sanitary napkin 20 is shown in FIG. 1 as viewed from its garment surface 20B. The body surface 20A of the sanitary napkin 20 is intended to be placed adjacent to the wearer's body when the sanitary napkin 20 is worn.

The sanitary napkin 20 has two centerlines, a longitudinal centerline L and a transverse centerline T. The term "longitudinal", as used herein, refers to a line, axis, or direction in the plane of the sanitary napkin 20 that is generally aligned with (e.g., approximately parallel to) a vertical plane which bisects a standing wearer into left and right body halves when the sanitary napkin 20 is worn. The terms "transverse" or "lateral" used herein, are interchangeable, and refer to a line, axis, or direction which lies within the plane of the sanitary napkin 20 that is generally perpendicular to the longitudinal direction.

FIG. 1 shows that the main body portion 21 of the sanitary napkin 20 comprises the portion of the sanitary napkin without the side wrapping elements. The main body portion 21 has two spaced apart longitudinal edges 22, two spaced apart transverse or end edges (or "ends") 24, which together form the periphery 26 of the main body portion of the sanitary napkin 20. The main body portion also has two end regions, which are designated first end region 28 and second end region 30. A central region 32 is disposed between the end regions 28 and 30. The end regions 28 and 30 extend outwardly from the edges of the central region 32 about 1/8 to about 1/3 of the length of the main body portion. A detailed description of a sanitary napkin having a central region 32 and the two end regions 28 and 30 is contained in U.S. Patent 4,690,680 issued to Higgins on September 1, 1987, the disclosure of which is incorporated herein by reference.

The main body portion of the sanitary napkin 20 can be of any thickness, including relatively thick, relatively thin, or even very thin. The embodiment of the sanitary napkin 20 shown in Figures 1-5 of the drawings is intended to be an example of a relatively thin sanitary napkin, preferably an "ultra-thin" sanitary napkin as described in U.S. Patents 4,950,264 and 5,009,653, both issued to Osborn. The sanitary napkin 20 shown should preferably also be relatively flexible, so that it is comfortable for the wearer. It should be understood that the sanitary napkin shown is merely one preferred embodiment, and that the present invention is not limited to absorbent articles of the type or having the specific configurations shown in the drawings.

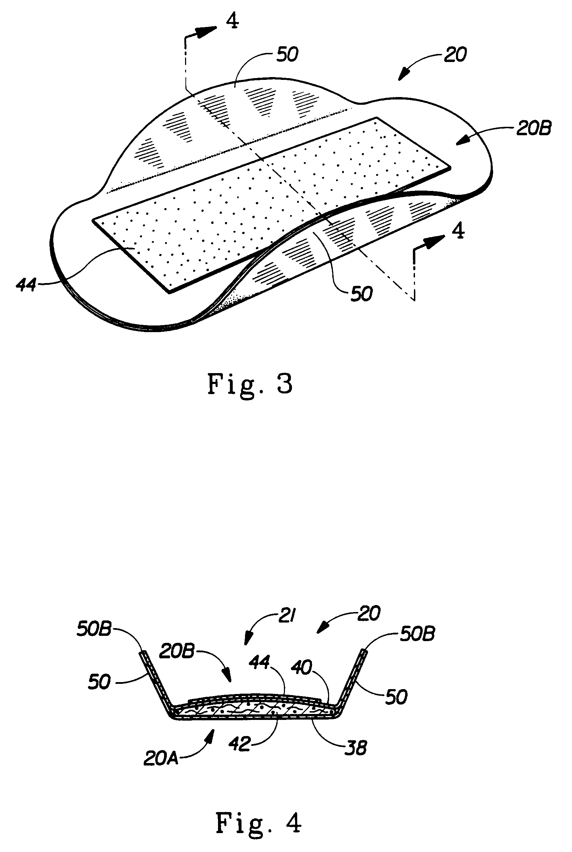

FIG. 4 shows the individual components of the main body portion 21 of the sanitary napkin 20 of the present invention. The main body portion 21 generally comprises at least three primary components. These include a liquid pervious topsheet 38, a liquid impervious backsheet 40, and an absorbent core 42 positioned between the topsheet 38 and the backsheet 40. There are occasions, however, when one or more of these components, such as the backsheet, can comprise part of the side wrapping elements described herein. The topsheet, the backsheet, and the absorbent core may be assembled in a variety of configurations known in the art (including so called "sandwich" products and "tube" products).

Several preferred sanitary napkin configurations are described generally in U.S. Patent 4,321,924, "Bordered Disposable Absorbent Article" issued to Ahr on March 30, 1982; U.S. Patent 4,425,130, "Compound Sanitary Napkin" issued to DesMarais on January 10, 1984; U.S. Patent 4,950,264, "Thin, Flexible Sanitary Napkin" issued to Osborn on August 21, 1990; U.S. Patent 5,308,346, "Elasticized Sanitary Napkin" issued to Sneller, et al. on May 3, 1994; PCT Patent Application WO 94/02096 entitled "Absorbent Articles Having Panty Covering Components That Naturally Wrap the Sides of Panties" with a priority of July 22, 1993, in the name of Lavash, et al.; and PCT Patent Application WO 95/07675 entitled "Absorbent Articles Having Panty Covering Components Comprising Extensible Web Materials Which Exhibit Elastics-Like Behavior" with a priority of September 17, 1993, in the name of Mansfield, et al. The main body portion 21 of the sanitary napkin may also be comprised of one or more extensible components such as those sanitary napkins, and the like described in PCT Publication Nos. WO 93/01785 and 93/01786, both published February 4, 1993.

The sanitary napkin 20, as shown in FIG. 4, is assembled in a sandwich construction in which the topsheet 38 and the backsheet 40 have length and width dimensions generally larger than those of the absorbent core 42. The topsheet 38 and the backsheet 40 extend beyond the edges of the absorbent core 42 to form portions of the periphery 26. The sanitary napkin 20 of the present invention comprises a pair of side wrapping elements 50 joined to the main body portion 21 that extend from proximal edges 52 laterally outward beyond the longitudinal side edges 22 of the main body portion 21 to their distal edges 54. The term "joined", as used herein, encompasses configurations in which an element is directly secured to another element by affixing the element directly to the other element; configurations in which the element is indirectly secured to the other element by affixing the element to intermediate member(s) which in turn are affixed to the other element; and configurations in which one element is integral with another element, i.e., one element is essentially part of the other element.

The side wrapping elements 50 can be of any suitable size and shape. The side wrapping elements 50 can be as large as any of the flaps described in the patents set out in the Background of the Invention, which are hereby incorporated by reference herein. Preferably, however, the distal edges 54 of the side wrapping elements preferably extend outward beyond the longitudinal side edges 22 of the main body portion 21, a distance of less than one-half the width of the main body portion so that they do not extend far enough outward beyond the side edges of the wearer's panties to cause any inconvenience to the wearer. The side wrapping elements 50 of the present invention preferably have the dimensions set forth for the panty covering components in the aforementioned PCT Patent Application WO 94/02096 and WO 95/07675 filed in the names of Lavash, et al. and Mansfield, et al.,. The side wrapping elements 50 can be made from any of the materials suitable for use in the construction of the main body portion 21 of the sanitary napkin. The side wrapping elements can comprise absorbent materials if the side wrapping elements are suitably sealed to prevent wicking of exudates from their distal edges 54. Preferably, however, the materials comprising the side wrapping elements are substantially non-absorbent.

The side wrapping elements 50 preferably have a resistance to edge compression so that they will fold rather than crumple when placed in a wearer's panties and subjected to the forces associated with wearing the sanitary napkin. The term "resistance to edge compression" refers to how substantial the material that comprises the side wrapping elements 50 is. Specifically, edge compression refers to the tendency of the side wrapping elements 50 to buckle when the side wrapping elements are extended to form a planar extension and forces are applied to the distal edge 54 of the side wrapping element by a plate oriented perpendicular to the plane of the side wrapping elements 50. This property is important because if the side wrapping elements 50 are insubstantial, they will bunch up when forces are applied to the side wrapping elements by the wearer's panty elastics or by the wearer's thighs during wear. The side wrapping elements 50 should, of course, not be so stiff that they are uncomfortable to the wearer.

In the preferred embodiment of the present invention shown in FIGS. 1-5, the side wrapping elements 50 are provided with first regions 50A comprising softened, flexible and preferably extensible regions (or "portions") of the side wrapping elements 50 and a second region (or "distal edge portion") 50B that is less soft, less flexible (i.e., stiffer), and preferably less extensible than the first regions 50A. The side wrapping elements 50 are pre-disposed to fold around the side edges of the crotch region of the wearer's undergarments. The second region 50B of the side wrapping elements 50 maintains a line of tension along the distal edges 54 of the side wrapping elements 50 that facilitates automatic flipping of the side wrapping elements 50 around the side edges of the wearer's undergarment when the undergarment is raised into position.

The first and second regions 50A and 50B of the side wrapping elements can have many possible shapes and arrangements. The first region 50A may comprise a single region, or a plurality of regions or portions of the side wrapping elements. If the first region 50A comprises a plurality of portions of the side wrapping elements, these portions of the side wrapping elements may be interconnected or they may be spaced apart. Regardless of the arrangement and shape used, the first regions 50A are preferably positioned inboard of the distal edges 54 of the side wrapping elements. The first regions 50A are preferably far enough inboard of the distal edges 54 of the side wrapping elements that the side wrapping elements 50 each have a second region 50B that comprises a band of substantially inextensible, unsoftened material that is disposed along at least a portion of, and preferably along all of the distal edge 54 of the side wrapping elements. This allows the distal edges 54 of the side wrapping elements to function in effect like a hoop that retains a constant length which assists the side wrapping elements 50 in automatically wrapping around the edges of the wearer's panty crotch. This automatic wrapping action is facilitated when the main body portion of the sanitary napkin is placed in a wearer's panties and the main body portion assumes an upwardly arched configuration along its longitudinal centerline when the panties are pulled into contact with the wearer's body. The arching of the main body portion places tension on the distal edges 54 of the side wrapping elements 50. This causes the side wrapping elements 50 to fold under the panties since the side wrapping elements will tend to assume a configuration that is closest to a straight line (between the ends of the arching main body portion) when they are under tension.

In the preferred embodiment shown in FIGS. 1-5, the first regions 50A comprise a plurality of spaced apart triangularly-shaped regions of the side wrapping elements 50 that are softened and provided with increased flexibility. The first regions 50A are oriented so that the apex (that is, the narrowest portion) of the triangularly-shaped areas is closest to the proximal edges 52 of the side wrapping elements, and the bases (or widest portions) of the triangularly-shaped areas are closer to the distal edges 54 of the side wrapping elements. This allows for greater reduction of the stresses in the side wrapping elements near the distal edges of the side wrapping elements. The reduction of stresses is, thus, at those locations where the stresses are the greatest when the side wrapping elements 50 are folded around the crotch region of a panty.

The side wrapping elements 50 can be provided with first and second regions 50A and 50B in a non-limiting number of different manners. The side wrapping elements 50 may, for example, comprise a substantially inextensible material that is provided with mechanically softened or extensible regions inboard of the distal edges 54 of the side wrapping elements for the first regions 50A. The mechanically softened or extensible regions can be created in any suitable manner, including but not limited to mechanically straining, corrugating, "ring rolling", heating and deforming, subjecting to compression between mating plates, and the like.

In other alternative embodiments, the different regions of the side wrapping elements can be provided by forming the side wrapping elements 50 out of materials having different properties. For example, the side wrapping elements 50 can be comprised of a laminate of an extensible material and a inextensible material. In such a embodiment, the inextensible material can be provided in the configuration of the side wrapping elements 50, but have holes cut out where the first regions 50A are to be located, which is then laminated to the extensible material. Alternatively, the side wrapping elements 50 can comprise a generally extensible material that has a strip of inextensible material attached along or wrapped around the portion of the extensible material that will form the distal edge 54 of the side wrapping element.

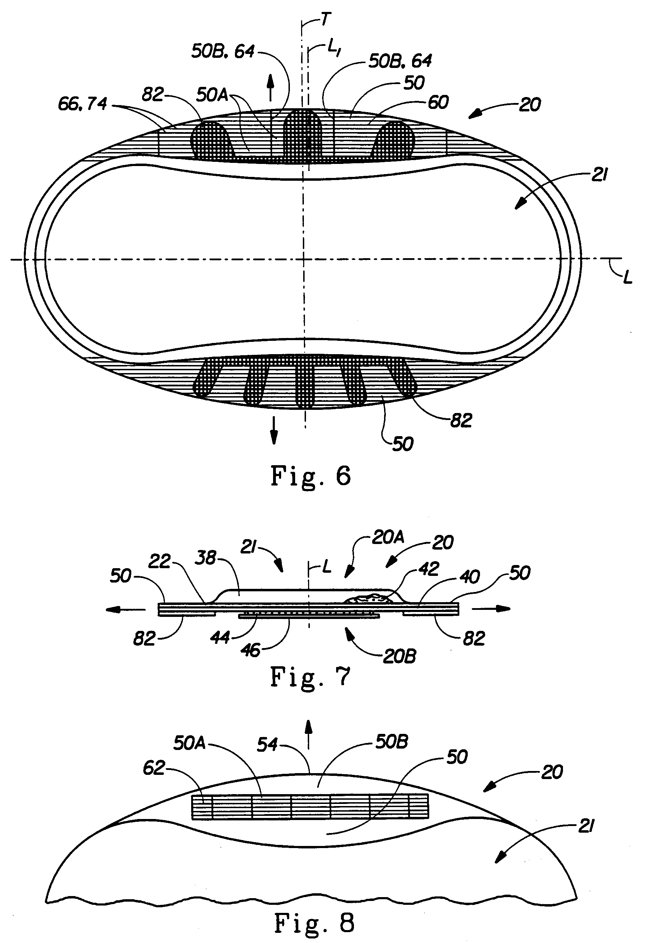

The embodiment shown in FIGS. 1-5 has first regions 50A that are preferably either formed by ring rolling (or pre-corrugating) or by forming a strainable network in five separate regions of each of the side wrapping elements 50. The first regions 50A preferably have corrugations or deformations with ridges (or fold lines) 58 that are generally oriented in the longitudinal direction. The side wrapping element 50 may fold through a large number of combinations of these longitudinally-oriented ridges or fold lines. This allows the side wrapping elements 50 to accommodate panties having different sizes and shapes. Suitable methods for ring rolling are described in U.S. Patent 4,107,364 issued to Sisson on August 15, 1978, U.S. Patent 4,834,741 issued to Sabee on May 30, 1989, U.S. Patent 5,143,679 issued to Gerald M. Weber, et al. on September 1, 1992, U.S. Patent 5,156,793 issued to Kenneth B. Buell, et al. on October 20, 1992, and U.S. Patent 5,167,897 issued to Gerald M. Weber, et al. on December 1, 1992. The formation of a strainable network into a web material is described below in conjunction with the embodiments shown in FIGS. 6-8.

The first regions 50A in this embodiment are preferably extensible in a direction perpendicular to the fold lines 58 of the corrugations or deformations. The first regions 50A are preferably primarily extensible in the transverse direction. The phrase "primarily extensible in the transverse direction" means that the first regions 50A are preferably more extensible in the transverse direction than in the longitudinal direction. However, the first regions 50A can also be primarily extensible in the longitudinal direction, or in any direction between the longitudinal direction and the transverse direction. If there is more than one first region 50A, the extensibility of different first regions 50A can all be in the same direction. Alternatively, one or more of the first regions 50A may have an extensibility in a different direction.

The garment surface 20B of the sanitary napkin 20 may include, and preferably does include, fasteners for attaching the sanitary napkin to the wearer's undergarments. Figure 2 shows the central pad fastener 44 which is adapted to secure the main body portion 21 of the sanitary napkin to the crotch region of an undergarment. Fasteners comprising adhesives have been found to work well for this purpose, with pressure sensitive adhesives being preferred. Before the sanitary napkin 20 is placed in use, if an adhesive fastener is used, the adhesive is typically covered with a removable cover strip or release liner 46 in order to keep the adhesive from sticking to a surface other than the crotch portion of the panty prior to use. Suitable release liners are described in the U.S. Patent 4,917,697.

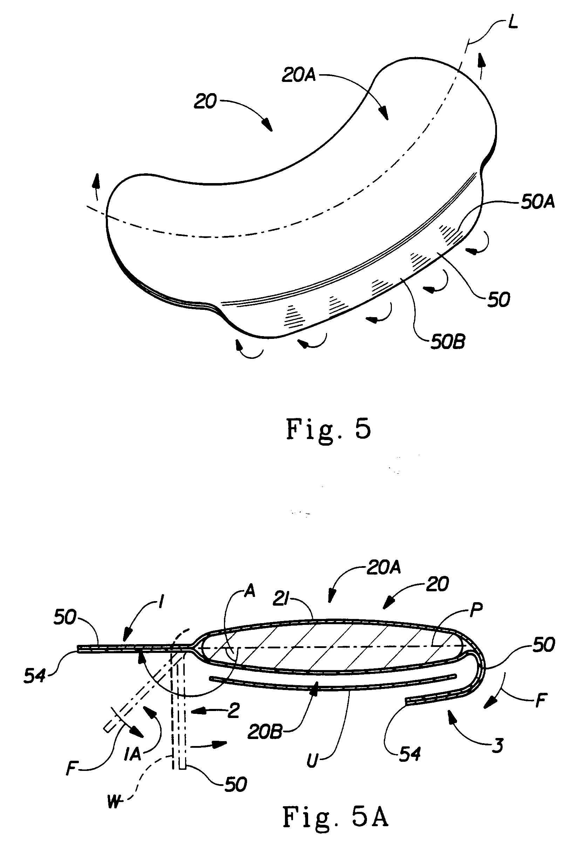

The sanitary napkin 20 of the present invention is used by removing any release liner and thereafter placing the sanitary napkin 20 in a panty so that the adhesive (or other fastener) 44 contacts the inside surface of the crotch region of the panty and maintains the sanitary napkin in position within the panty during use. The side wrapping elements 50 automatically wrap around the sides of the wearer's panties when the main body portion 21 of the sanitary napkin is placed in a wearer's panties and the main body portion assumes an upwardly arched configuration along its longitudinal centerline as shown in FIG. 5 when the panties are pulled into contact with the wearer's body.

FIG. 5A shows that the side wrapping elements 50 are preferably capable of assuming three distinct positions when the sanitary napkin 20 is placed in a wearer's undergarments U during wear. These comprise: (a) a first stable position in which the side wrapping elements 50 extend generally outward from the main body portion 21; (b) an unstable position in which they are partially folded toward the garment-facing side of the main body portion; and, (c) a second stable position in which said side wrapping elements form an acute angle with the garment-facing side of said main body portion.

The first stable position can be a position such as that designated position (1) in which the side wrapping elements 50 extend straight out from the sides of the main body portion 21. That is, the side wrapping elements form an angle of 180° with a plane, P, which passes through a section of the main body portion taken along the transverse centerline of the sanitary napkin. (It is understood, however, that the main body portion, per se, will typically be in a curved or folded overall configuration, rather than planar configuration.) In alternative embodiments, the side wrapping elements 50 may have a first stable position such as that designated position (1A) where the side wrapping elements 50 initially form an obtuse angle (A) relative to the plane, P, of the section of the main body portion taken along the transverse centerline. The embodiment shown in FIGS. 1-5 can serve as an example of an embodiment in which the side wrapping elements 50 may form an obtuse angle with respect to the plane of the main body portion in their first stable position. When forces F are applied to the side wrapping elements 50 in the direction of the arrows in FIG. 5A (such as by the wearer's thighs W), the side wrapping elements 50 move to an unstable position (2). The side wrapping elements 50 are typically in an unstable position at all locations between positions (1A) and (3). An unstable position may, for example, be approximately at a 90° fold angle relative to the plane P. The side wrapping elements 50 preferably then have a tendency to flip to their second stable position, position (3). In their second stable position, the side wrapping elements 50 fold more toward the garment-facing side of the main body portion, and preferably form an acute angle with the garment-facing side of the main body portion. The side wrapping elements 50 will typically fold smoothly through the three positions referred to herein so that the precise place where one position ends and another begins may blend into one another. It should also be understood that FIG. 5A is primarily presented for purposes of illustration of the concepts referred to herein. The present invention is, therefore, not intended to be limited to absorbent articles having side wrapping elements 50 that assume the exact positions shown in FIG. 5A.

The operation of the side wrapping elements 50 is distinguishable in several respects from that of conventional side flaps. Placing a sanitary napkin having conventional flaps in a pair of panties and pulling up the panties will not consistently provide the automatic sustained wraparound feature of the present invention. There are several reasons for this. Conventional flaps do not have a distal edge that is maintained in tension that facilitates automatic flipping of such flaps around the side edges of a wearer's undergarment when the undergarment is pulled into its in-use condition. Conventional flaps are also not provided with resistance to edge compression so that they will tend to crumple in use, particularly when the wearer's thighs exert compressive forces on the flaps. Conventional flaps also do not retain a high degree of their fold, so that in cases where conventional flaps do wrap around the panties, they will not consistently stay wrapped. By contrast, once the side wrapping elements of the present invention are folded, the upward arching of the main body portion tends to increase tension in the distal edge, thereby locking the pad in its in-use condition. In addition, conventionally-sized flaps will have excess flap material that hangs down underneath the panties during wear. This material can move around excessively underneath the panties. The side wrapping elements of the present invention, on the other hand, have a span that is ideally just wide enough to wrap around the elastic-containing edges of the panties, but no wider, avoiding the problems associated with excess flap material.

The sanitary napkin 20 of the present invention can be provided with still other features to assist in positioning the side wrapping elements 50 in the wearer's panties. The side wrapping elements 50 shown in FIGS. 1-5 are initially folded toward the garment-facing side 20B of the sanitary napkin and over the top of the release paper 46 that protects the securement adhesive 44 on the back of the sanitary napkin. FIG. 2 shows that if the side wrapping elements 50 and the release paper 46 are properly sized in relation to one another, stripping away the release paper 46 can serve to automatically unfold the side wrapping elements 50 from the back of the sanitary napkin and cause the side wrapping elements 50 to project generally outwardly in the desired orientation for installation of the sanitary napkin in the undergarment. FIGS. 3 and 4 show that when the release paper 44 is removed, there will be a tendency for the side wrapping elements 50 to automatically return part of the way to their original folded over condition because the material comprising the side wrapping elements 50 will retain memory of the original folded over condition due to the residual stresses resulting from the folding. The sanitary napkin is then placed into the wearer's panties and the panties are pulled into the desired wearing position. Pulling the panties up against the wearer's body will force the main body portion 21 of the sanitary napkin into an arc. This will cause the inextensible perimeter of the side wrapping elements to experience tension and will cause the side wrapping elements 50 to collapse around the panty crotch.

Another variable which can be used to assist in positioning of the side wrapping elements 50 in a preferred orientation for installation in the wearer's panties is the packaging of the sanitary napkin in an individual wrapper. For example, a sanitary napkin in the embodiment shown in FIGS. 1-5 can be tri-folded and placed in an individual package so that it can be kept clean while it is carried by the user in her purse. If the sanitary napkin is tri-folded inwardly over the backsheet, this will make the side wrapping elements 50 pre-disposed to fold toward the backsheet when they are removed from the package and placed in the wearer's undergarment. That is, the folding of the sanitary napkin might be used to bias the side wrapping elements 50 into a configuration that would make its final position easier to achieve without any conscious act on the part of the user. It should also be understood that this feature and the preceding additional feature can be employed in conjunction with any of the other embodiments described below.

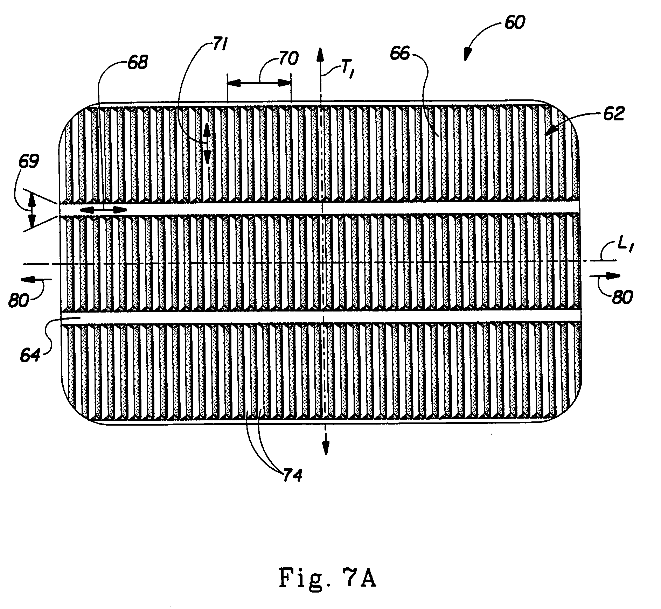

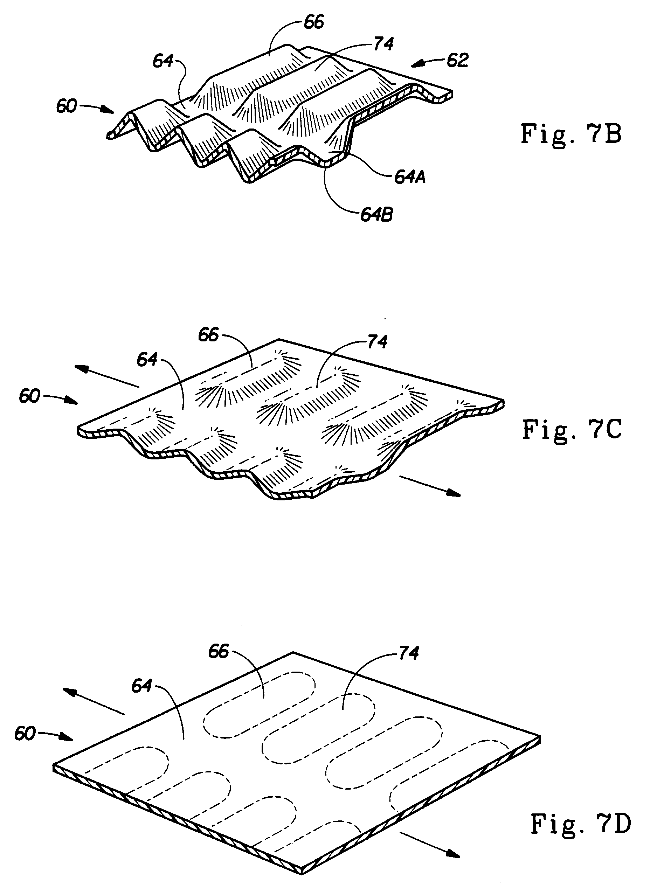

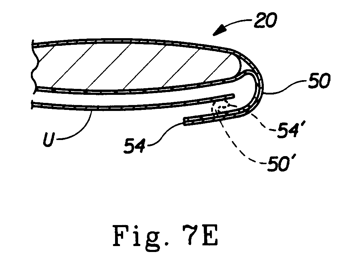

FIGS. 6 and 7 show a second preferred embodiment of a sanitary napkin 20 of the present invention. In this embodiment, the side wrapping elements 50 comprise a first region 50A that is distributed throughout the entirety of the side wrapping elements 50. The side wrapping elements 50 are primarily extensible in the transverse direction (shown by the arrows). The side wrapping elements 50 also comprise less extensible second regions 50B. The second regions 50B are disposed throughout and between portions of the first region 50A. The less extensible second regions 50B maintain the side wrapping elements 50 in tension when the first region 50A of the side wrapping element 50 is extended laterally outward. This facilitates the flipping of the side wrapping elements around the side edges of the wearer's undergarment.

The side wrapping elements 50 shown in FIGS. 6 and 7 preferably comprise a web material 60 having a network 62 of strainable regions formed therein that exhibits an elastic-like behavior in the direction of elongation without the use of added elastic materials. This web material may be referred to herein as a "strainable web material" for brevity. In the embodiment shown in FIGS. 6 and 7, the strainable web material 60 comprises a laminate of an extensible nonwoven material, an apertured formed film, and a polyethylene film backsheet material. The extensible nonwoven material should be oriented so that its extensibility is in the lateral direction. The apertured formed film is preferably an apertured film known as DRI-WEAVE which is used as a topsheet on sanitary napkins manufactured by the Procter & Gamble Company, Cincinnati, Ohio under U.S. Patents 4,342,314 issued to Radel, et al. and 4,463,045 issued to Ahr, et al. The polyethylene backsheet material can be an ordinary backsheet material, and preferably has a caliper of about 1 mil.

FIGS. 7A and 7B are enlarged views of a web material with a strainable network therein such as that shown in FIGS. 6 and 7. FIGS. 7A and 7B are simplified in that they illustrate the concept of the strainable network in one of the components of the laminate, the film backsheet material. The web material 60 will be described accordingly in FIGS. 7A and 7B, although it is understood that in the embodiment shown in FIGS. 6 and 7, the web material comprises two additional components. The strainable web material 60 is shown in FIGS. 7A and 7B in its substantially untensioned condition. The strainable web material 60 has a longitudinal centerline, L₁, and a lateral centerline, T₁. The lateral centerline, T₁, is generally perpendicular to the longitudinal centerline, L₁.

FIGS. 7A and 7B show that the strainable web material 60 includes a strainable network 62 of at least two distinct and dissimilar regions. The term "strainable network", as used herein, refers to an interconnected and interrelated group of regions which are able to be extended to some useful degree in a predetermined direction. The two distinct regions provide the strainable web material 60 with a first elastic-like, relatively low resistive force stage and a second relatively high resistive force stage. The strainable network 62 described herein is formed into the web material. As used herein, the term "formed" refers to the creation of a desired structure or geometry upon the web material that will substantially retain the desired structure or geometry when it is not subjected to any externally applied elongations or forces. Suitable methods for forming a strainable network described herein into a material include, but are not limited to embossing by mating plates or rolls, thermoforming, high pressure hydraulic forming, or casting.

FIGS. 7A and 7B show that the two distinct regions of the strainable network 62 include at least a first region 64 and a second region 66. The first region 64 will exhibit resistive forces in response to an applied axial elongation before a substantial portion of the second region 66 develops significant resistive forces to the applied elongation. The second region 66 has a surface-pathlength which is greater than that of the first region 64. The surface-pathlengths are measured substantially parallel to the predetermined axis while the material is in an untensioned condition. The second region 66 includes one or more deformations 74 which extend beyond the plane of the first region 64. The first and second regions each have a first surface and an opposing second surface. In the preferred embodiment shown in FIGS. 7A and 7B, the strainable network 62 includes a plurality of first regions 64 and a plurality of second regions 66. In the preferred embodiment shown in FIGS. 7A and 7B, the first regions 64 are substantially planar regions. That is, the material within the first region 64 is in substantially the same condition before and after the formation step undergone by strainable web material. The second regions 66 include a plurality of continuous, interconnected, deformations 74 which extends alternately beyond the plane of both the first and second surfaces (64A and 64B, respectively) of the first region 64. In other embodiments, the deformations 74 may extend beyond the plane of only one of either the first or the second surfaces of the first region.

The first regions 64 have a first axis 68 and a second axis 69, wherein the first axis 68 is preferably longer than the second axis 69. The first axis 68 of the first region 64 is substantially parallel to the longitudinal axis, L₁, of the strainable web material 60 while the second axis 69 is substantially parallel to the transverse axis, T₁, of the strainable web material 60. The second regions 66 also have a first axis 70 and a second axis 71. The first axis 70 of the second region 66 is substantially parallel to the longitudinal axis L₁ of the strainable web material 60, while the second axis 71 is substantially parallel to the transverse axis T₁ of the strainable web material 60. In the preferred embodiment of FIG. 7A, the first regions 64 and the second regions 66 are substantially linear, extending continuously in a direction substantially parallel to the longitudinal axis L₁ of the strainable web material. In the preferred embodiment shown in FIGS. 6 and 7, the longitudinal centerline L₁ of the strainable web material 60 is generally aligned with the transverse centerline of the sanitary napkin 20. In other embodiments, however, the longitudinal centerline L₁ of the web material can be oriented in other directions, depending on the direction of extensibility desired.

The strainable web material 60 preferably comprises at least one component which is a formed polymeric film. The strainable web material 60 can be made of a base film material that has a relatively low extensibility under the forces the sanitary napkin is normally subjected to when worn. When formed into the strainable web material as described 60 herein, however, the base material will be extensible under the forces the sanitary napkin is normally subjected to when worn. The strainable web material 60 is preferably comprised substantially of linear low density polyethylene (LLDPE). The strainable web material 60 may also be comprised of other polyolefins such as polyethylenes, including low density polyethylene (LDPE), ultra low density polyethylene (ULDPE), high density polyethylene (HDPE), or polypropylene and blends thereof with the above and other materials. Examples of other suitable polymeric materials which may also be used include, but are not limited to polyester, polyurethanes, compostable or biodegradable polymers, heat shrink polymers, thermoplastic elastomers, and breathable polymeric structures.

FIGS. 7B, C and D show the manner in which the strainable web material 60 exhibits at least two significantly different stages of controlled resistive force to elongation when subjected to an applied elongation in a direction parallel to a predetermined axis. The strainable web material 60 exhibits first resistive forces to the applied elongation (which develop between the stage shown in FIG. 7B and the stage shown in FIG. 7C). The first resistive forces occur until the elongation of the web is sufficient to cause a substantial portion of the second region 66, to enter the plane of applied elongation, as shown in FIG. 7C. After the strainable web material 60 reaches the stage shown in Fig. 7C, it exhibits a second stage of resistive forces to further elongation (as illustrated by FIG. 7D). Typically, when used in the side wrapping elements 50 of the present invention, the web material will be within the first stage of resistance to elongation so the various portions of the strainable web material 60 will only extend to the stage shown in FIG. 7C and adjust so as to relax back to the stage shown in FIG. 7B.

The depth and number of deformations 74 in the strainable web material 60 can be varied to control the applied force or elongation required to extend the web material used in the side wrapping elements 50. In one preferred embodiment, the deformations 74 are formed by two rigid plates having outer dimension of 127 mm by 304.8 mm by 19.04 mm (5.0" by 12" by 0.75"). On one surface of each plate are a series of meshing teeth which are substantially triangular in cross section and measure 0.030" at their bases and taper to a vertex with a radius of 0.2032 mm (0.008") at the top. The centerlines of the teeth are spaced evenly and at 0.762 mm (0.030") increments. On the "toothed" side of one plate, a series of grooves are cut which are parallel to each other and perpendicular to the evenly spaced teeth. These grooves measure 0.7874 mm (0.031") wide and are continuous over the entire length of the plate, and are spaced at a distance of 6.35 mm (0.25") on center. These grooves correspond to the undeformed regions of the deformed web of material. The preferred web material is placed between the plates in a hydraulic press having platens larger than the plates to evenly distribute pressure. The plates are compressed under a load of at least 1816 kg (4,000 pounds). The formed web material is then removed from between the plates. The available stretch or elongation is increased if for a given number of deformations, the height or degree of deformation imparted on the deformations is increased. Similarly, the available stretch or elongation is increased if for a given height or degree of deformation, the number or frequency of deformations is increased.

The embodiment shown in FIGS. 6 and 7 uses controlled stresses in the different regions of the strainable web material 60 to cause the side wrapping elements 50 to curl around to enclose the edges of the panty. This is desirable, as it may offer performance like a conventional wing but without wing or flap adhesives. FIG. 7E shows one configuration the side wrapping element 50 might take. However, the stress which causes this curling to occur may also cause the side wrapping element 50 to occasionally curl too much (as shown by the side wrapping element 50' in FIG. 7E) and slip away from the panty, losing coverage of the edges of the panty. Therefore, the side wrapping elements 50 must also resist the tendency to curl excessively. This may be controlled by the stiffness of the materials forming the side wrapping elements 50. One way to control the stiffness of the side wrapping elements 50 to prevent curling is to add a reinforcing element such as a scrim 82 to the side wrapping elements 50.

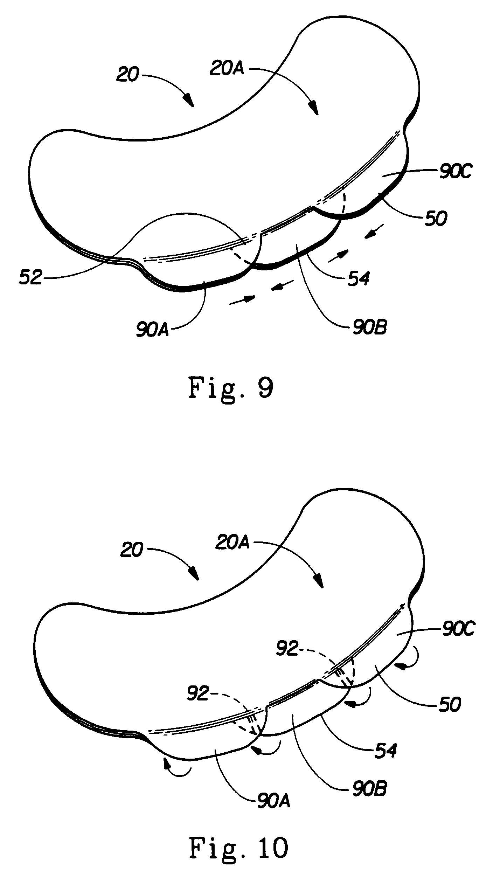

FIG. 8 shows an alternative embodiment of the sanitary napkin shown in FIGS. 6 and 7. The sanitary napkin shown in FIG. 8 has a strainable network 62 formed into only a portion of the area of its side wrapping element. The strainable network 62 is formed so that a portion of the side wrapping element 50 along the distal edge 54 of the side wrapping element is not "formed". The embodiment shown in FIG. 8 has the advantage of increasing the tension along the distal edge 54 of the side wrapping element 50 to facilitate the automatic folding of the side wrapping elements 50 when the undergarment is pulled into its use condition. This may also make the distal edge 54 stiffer, reducing the tendency for the side wrapping element 50 to curl under the wearer's undergarment.

FIGS. 9 and 10 show a third basic embodiment of the sanitary napkin of the present invention. The embodiment shown in FIGS. 9 and 10 has side wrapping elements 50 that comprise a plurality of spaced apart regions or components that are gathered longitudinally inward along the distal edges 54 of the side wrapping elements 50 as shown in FIG. 9 and joined together. The spaced apart regions or components are shown in FIGS. 9 and 10 as comprising separate overlapping side wrapping element components 90A, 90B, and 90C. The side wrapping element components 90A, 90B, and 90C are gathered longitudinally inward along the distal edges 54 of the side wrapping elements 50 as shown in FIG. 9 and joined together by two seams 92 as shown in FIG. 10. This embodiment eliminates any bunching or puckering that would ordinarily be associated with gathering such structures using elastic strands.

In alternative versions of this embodiment, instead of comprising overlapping components, the regions of components of the side wrapping elements 50 that are gathered can be separate components that are non-overlapping, or they can merely be different regions of a single component side wrapping element. The regions or components of the side wrapping elements 50 can be gathered inward either before or after the side wrapping elements 50 are joined to the main body portion 21 of the sanitary napkin. Gathering of the side wrapping elements 50 may, however, be somewhat easier to accomplish before the side wrapping elements are joined to the main body portion 21 when the side wrapping elements 50 are in the form of a continuous web.

The gathering inward of the regions or components 90A, B and C of the side wrapping elements 50 maintains a line of tension along the distal edges 54 of the side wrapping elements 50. This line of tension facilitates the flipping of the side wrapping elements 50 under the wearer's undergarment. This flipping action is further facilitated when the main body portion 21 of the sanitary napkin is placed in a wearer's panties and the main body portion 21 assumes a curved configuration when the panties are pulled up into contact with the wearer's body. In an alternative version of this third embodiment, the spaced apart regions or components of the side wrapping elements can be gathered longitudinally inward along the proximal edges 52 of the side wrapping elements to create a different effect.

There are numerous possible alternative embodiments and variations of the present invention shown in the preceding drawing figures. For example, the side wrapping elements are preferably mirror images of each other, and are symmetrical about the longitudinal centerline. However, it should be understood that the shape and location of the side wrapping elements described herein are those of a preferred embodiment, and other embodiments are also possible. In addition, while the side wrapping elements 50 are shown as extending from each longitudinal edges of the main body portion, in other alternative embodiments, there may only be one side wrapping element extending from one of the edges of the main body portion. The side wrapping elements 50 may be offset along the longitudinal centerline more towards one end edge of the main body portion than the other. In still other embodiments, the side wrapping elements 50 may be separate elements that are joined underneath to the main body portion 21 of the sanitary napkin inboard of the longitudinal side edges 22 of the main body portion. The side wrapping elements 50, in such a case, may be otherwise unattached to the garment-facing side of the main body portion 21 of the sanitary napkin 20 between the points of attachment and the longitudinal side edges 22 of the main body portion.

The present invention is also applicable to other types of absorbent articles worn in the crotch region of an undergarment such as pantiliners and incontinence articles. The terms "panty liner" or "pantiliner" refer to absorbent articles that are less bulky than sanitary napkins which are generally worn by women between their menstrual periods. Examples of suitable absorbent articles in the form of pantiliners that can be provided with the side wrapping elements described herein are disclosed in U.S. Patent 4,738,676 entitled "Pantiliner" issued to Osborn on April 19, 1988.

The term "incontinence article" refers to pads, undergarments (pads held in place by a suspension system of same type, such as a belt, or the like), inserts for absorbent articles, capacity boosters for absorbent articles, briefs, bed pads, and the like, regardless of whether they are worn by adults or other incontinent persons. Examples of suitable incontinence articles that can be provided with the side wrapping elements described herein are disclosed in U.S. Patent 5,300,054 issued to Feist, et al. on April 5, 1994 and U.S. Patent 5,304,161 issued to Noel, et al. April 19, 1994.

While particular embodiments of the present invention have been illustrated and described, it would be obvious to those skilled in the art that various other changes and modifications can be made without departing from the invention.

## Claims

1. An absorbent article (20) for wearing in a crotch region of an undergarment, said absorbent article (20) having a longitudinal dimension extending in a longitudinal direction (L) and a transverse dimension extending in a transverse direction (T), said absorbent article (20) comprising:
a main body portion (21) comprising an absorbent core (42), said main body portion (21) having a body-facing side (20A), a garment-facing side (20B), and a pair of longitudinal side edges (22); and
a pair of side wrapping elements (50) joined to said main body portion and extending laterally outward beyond the longitudinal side edges of said main body portion to distal edges (54), characterized in that said side wrapping elements (50) are pre-disposed to wrap around an edge of the crotch region of the wearer's undergarment when the main body portion (21) is placed into an upwardly arcuate configuration, said side wrapping elements (50) comprising a first region (50A) that is free of elastic strands and extensible primarily in the transverse direction (T) and a less extensible second region (50B) disposed laterally outward of said first region (50A) that maintains said side wrapping elements (50) in tension when said first region (50A) is extended laterally outward.

2. The absorbent article (20) of Claim 1 wherein said side wrapping elements (50) are capable of assuming (a) a first stable position in which they generally extend outward from said main body portion (21); (b) an unstable position when they are partially folded toward the garment-facing side (20B) of the main body portion (21); and (c) a second stable position in which said side wrapping elements (50) form an acute angle with the garment-facing side (20B) of said main body portion (21).

3. The absorbent article (20) of Claim 1 wherein portions of said less extensible region are also disposed between portions of said first region (50A).

4. The absorbent article (20) of Claim 3 wherein said side wrapping elements (50) comprise a web material (60) having a strainable network (62) of distinct regions which exhibits at least two-stages of controlled resistive forces to an applied axial elongation along at least one predetermined axis when subjected to applied axial elongation in a direction parallel to said predetermined axis, said distinct regions comprising at least a first region (64) and a second region (66), said first region (64) having a surface-pathlength that is less than that of said second region (66) when measured parallel to said predetermined axis while said web material (60) is in an untensioned condition.

5. The absorbent article (20) of Claim 4 wherein said web (60) has a longitudinal axis (L1) aligned with the transverse centerline (T) of the absorbent article (20) and said network (62) of said web material (60) comprises a formed portion of a base material, and said first region (64) of said network (62) comprises a plurality of substantially planar, linear regions that are aligned with the longitudinal axis (L1) of said web material (60) and said second regions (66) comprises a plurality of deformations which are oriented substantially perpendicular to said longitudinal axis (L1) of said web material (60).

6. The absorbent article (20) of Claim 5 wherein at least portions of said web material (60) are reinforced to prevent curling of the distal edge (54) of said side wrapping elements (50).

7. The absorbent article (20) of Claim 6 wherein said portions of said side wrapping elements (50) are reinforced with a scrim.

8. An absorbent article (20) according to claim 1 wherein said first region (50A) comprises softened, flexible portion of said side wrapping element (50), and said second region (50B) is less soft and less flexible than said first region (50A).

9. The absorbent article (20) of Claim 8 wherein said second region (50B) is disposed along the entire distal edge (54) of said side wrapping element (50).

10. The absorbent article (20) of Claim 8 wherein said second region (50B) is substantially inextensible.

11. The absorbent article (20) if Claim 8 wherein said first region (50A) comprises a plurality of spaced apart softened, flexible portions.

12. The absorbent article (20) of Claim 8 wherein said first region (50A) is extensible, and said extensibility is primarily in the transverse direction (T).

13. The absorbent article (20) of Claim 12 wherein said first regions (50A) have a dimension measured in the longitudinal direction (L) adjacent the distal edge (54) of said side wrapping elements (50) and a dimension measured at the portion of said side wrapping elements (50) adjacent the proximal edge (52) of said side wrapping elements (50) and said dimension measured adjacent the distal edge (54) of said side wrapping elements (50) is greater than the dimension adjacent the proximal edge (52) of said side wrapping elements (50).

14. The absorbent article (20) of Claim 13 wherein said first regions (50A) of said side wrapping elements (50) are triangular-shaped.

15. The absorbent article (20) of Claim 8 wherein said first portions (50A) of said side wrapping elements (50) comprise a material selected from the group consisting of the following: an extensible material; a mechanically modified material; a strained material; a corrugated material; a ring rolled material; a permanently deformed material; and, a heated and stretched material.

16. An absorbent article (20) according to claim 1 wherein at least one of said side wrapping elements (50) comprises a plurality of regions that are gathered longitudinally inward along one of said distal or proximal edges (52, 54) and joined together in a gathered condition.

17. The absorbent article (20) of Claim 16 wherein said regions are gathered inward along the distal edge (54) of the side wrapping element (50).

18. The absorbent article (20) of Claim 16 wherein said regions are gathered inward along the proximal edge (52) of the side wrapping element (50).

19. The absorbent article (20) of Claim 16 wherein the regions of said side wrapping element (50) comprise integral portions of a unitary structure.

20. The absorbent article (20) of Claim 16 wherein the regions of said side wrapping element (50) comprise separate side wrapping element components.

21. The absorbent article (20) of Claim 20 wherein said separate side wrapping element components are joined together by a seam.

## Patentansprüche

1. Ein absorbierender Artikel (20) zum Tragen in einem Schrittbereich eines Unterwäschestücks, wobei der genannte absorbierende Artikel (20) eine Längsdimension, welche sich in einer Längsrichtung (L) erstreckt, und eine Querdimension, welche sich in einer Querrichtung (T) erstreckt, aufweist, wobei der genannte absorbierende Artikel (20) umfaßt:
einen Hauptkörperabschnitt (21), welcher einen absorbierenden Kern (42) umfaßt, wobei der genannte Hauptkörperabschnitt (21) eine dem Körper zugewandte Seite (20A), eine der Kleidung zugewandte Seite (20B) und ein Paar Längsseitenränder (22) aufweist; und
ein Paar seitenumhüllende Elemente (50), welche mit dem genannten Hauptkörperabschnitt verbunden sind und sich seitlich auswärts über die Längsseitenränder des genannten Hauptkörperabschnitts hinaus zu distalen Rändern (54) erstrecken, dadurch gekennzeichnet, daß die genannten seitenumhüllenden Elemente (50) vor-festgelegt sind, um einen Rand des Schrittbereichs des Unterwäschestücks des Trägers zu umhüllen, wenn der Hauptkörperabschnitt (21) zu einer aufwärts bogenförmigen Konfiguration angeordnet ist, wobei die genannten seitenumhüllenden Elemente (50) einen ersten Bereich (50A), welcher frei von elastischen Litzen ist und hauptsächlich in der Querrichtung (T) verlängerbar ist, und einen weniger verlängerbaren zweiten Bereich (50B), welcher seitlich auswärts vom genannten ersten Bereich (50A) angeordnet ist, welcher die genannten seitenumhüllenden Elemente (50) unter Zugspannung hält, wenn der genannte erste Bereich (50A) seitlich auswärts verlängert wird, umfassen.

2. Der absorbierende Artikel (20) nach Anspruch 1, bei welchem die genannten seitenumhüllenden Elemente (50) imstande sind, einzunehmen (a) eine erste stabile Position, in welcher sie sich allgemein auswärts vom genannten Hauptkörperabschnitt (21) erstrecken; (b) eine unstabile Position, wenn sie teilweise gegen die der Kleidung zugewandte Seite (20B) des Hauptkörperabschnitts (21) gefaltet sind; und (c) eine zweite stabile Position, in welcher die genannten seitenumhüllenden Elemente (50) einen spitzen Winkel mit der der Kleidung zugewandten Seite (20B) des genannten Hauptkörperabschnitts (21) bilden.

3. Der absorbierende Artikel (20) nach Anspruch 1, bei welchem Abschnitte des genannten weniger verlängerbaren Bereichs ebenso zwischen Abschnitten des genannten ersten Bereichs (50A) angeordnet sind.

4. Der absorbierende Artikel (20) nach Anspruch 3, bei welchem die genannten seitenumhüllenden Elemente (50) ein Bahnenmaterial (60) mit einem dehnbaren Netzwerk (62) mit unterschiedlichen Bereichen umfassen, welches mindestens zwei Stufen von kontrollierten Widerstandskräften gegen eine aufgebrachte Achsialverlängerung entlang mindestens einer vorher festgelegten Achse aufweist, wenn es aufgebrachter Achsialverlängerung in einer Richtung parallel zur genannten vorher festgelegten Achse unterworfen wird, wobei die genannten unterschiedlichen Bereiche mindestens einen ersten Bereich (64) und einen zweiten Bereich (66) umfassen, wobei der genannte erste Bereich (64) eine Oberflächen-Weglänge aufweist, welche geringer ist als jene des genannten zweiten Bereichs (66), gemessen parallel zur genannten vorher festgelegten Achse, während das genannte Bahnenmaterial (60) sich in einem ungespannten Zustand befindet.

5. Der absorbierende Artikel (20) nach Anspruch 4, bei welchem die genannte Bahn (60) eine Längsachse (L1) aufweist, welche mit der Quermittellinie (T) des absorbierenden Artikels (20) achsial ausgerichtet ist und das genannte Netzwerk (62) des genannten Bahnenmaterials (60) einen geformten Abschnitt aus einem Trägermaterial umfaßt und der genannte erste Bereich (64) des genannten Netzwerks (62) eine Mehrzahl von im wesentlichen planen, linearen Bereichen umfaßt, welche mit der Längsachse (L1) des genannten Bahnenmaterials (60) achsial ausgerichtet sind, und der genannte zweite Bereich (66) eine Mehrzahl von Verformungen umfaßt, welche im wesentlichen lotrecht zur genannten Längsachse (L1) des genannten Bahnenmaterials (60) ausgerichtet sind.

6. Der absorbierende Artikel (20) nach Anspruch 5, bei welchem mindestens Abschnitte des genannten Bahnenmaterials (60) verstärkt sind, um ein Kräuseln des distalen Randes (54) der genannten seitenumhüllenden Elemente (50) zu verhindern.

7. Der absorbierende Artikel (20) nach Anspruch 6, bei welchem die genannten Abschnitte der genannten seitenumhüllenden Elemente (50) mit einer Gaze verstärkt sind.

8. Ein absorbierender Artikel (20) nach Anspruch 1, bei welchem der genannte erste Bereich (50A) einen weichgemachten, flexiblen Abschnitt der genannten seitenumhüllenden Elemente (50) umfaßt und der genannte zweite Bereich (50B) weniger weich und weniger flexibel als der genannte erste Bereich (50A) ist.

9. Der absorbierende Artikel (20) nach Anspruch 8, bei welchem der genannte zweite Bereich (50B) entlang dem gesamten distalen Rand (54) des genannten seitenumhüllenden Elements (50) angeordnet ist.

10. Der absorbierende Artikel (20) nach Anspruch 8, bei welchem der genannte zweite Bereich (50B) im wesentlichen unverlängerbar ist.

11. Der absorbierende Artikel (20) nach Anspruch 8, bei welchem der genannte erste Bereich (50A) eine Mehrzahl von voneinander beabstandeten weichgemachten, flexiblen Abschnitten aufweist.

12. Der absorbierende Artikel (20) nach Anspruch 8, bei welchem der genannte erste Bereich (50A) verlängerbar ist und die genannte Verlängerbarkeit hauptsächlich in der Querrichtung (T) ist.

13. Der absorbierende Artikel (20) nach Anspruch 12, bei welchem die genannten ersten Bereiche (50A) eine in der Längsrichtung (L) benachbart dem distalen Rand (54) der genannten seitenumhüllenden Elemente (50) gemessene Dimension und eine Dimension, welche an dem Abschnitt der genannten seitenumhüllenden Elemente (50), welcher benachbart dem proximalen Rand (52) der genannten seitenumhüllenden Elemente (50) ist, gemessen ist, aufweisen und die genannte benachbart zum distalen Rand (54) der genannten seitenumhüllenden Elemente (50) gemessene Dimension größer ist als die zum proximalen Rand (52) der genannten seitenumhüllenden Elemente (50) benachbarte Dimension.

14. Der absorbierende Artikel (20) nach Anspruch 13, bei welchem die genannten ersten Bereiche (50A) der genannten seitenumhüllenden Elemente (50) von dreieckiger Form sind.

15. Der absorbierende Artikel (20) nach Anspruch 8, bei welchem die genannten ersten Abschnitte (50A) der genannten seitenumhüllenden Elemente (50) ein Material umfassen, welches aus der Gruppe ausgewählt ist, welche aus dem Folgenden besteht: ein verlängerbares Material; ein mechanisch modifiziertes Material; ein gedehntes Material; ein geriffeltes Material; ein ringgewalztes Material; ein permanent verformtes Material; und ein erhitztes und gestrecktes Material.

16. Ein absorbierender Artikel (20) nach Anspruch 1, bei welchem mindestens eines der genannten seitenumhüllenden Elemente (50) eine Mehrzahl von Bereichen umfaßt, welche der Länge nach einwärts entlang einem des genannten distalen oder des genannten proximalen Randes (52,54) gerafft sind und in einem gerafften Zustand miteinander verbunden sind.

17. Der absorbierende Artikel (20) nach Anspruch 16, bei welchem die genannten Bereiche entlang dem distalen Rand (54) des seitenumhüllenden Elements (50) einwärts gerafft sind.

18. Der absorbierende Artikel (20) nach Anspruch 16, bei welchem die genannten Bereiche entlang dem proximalen Rand (52) des genannten seitenumhüllenden Elements (50) einwärts gerafft sind.

19. Der absorbierende Artikel (20) nach Anspruch 16, bei welchem die Bereiche des genannten seitenumhüllenden Elements (50) integrale Abschnitte einer einstückigen Struktur umfassen.

20. Der absorbierende Artikel (20) nach Anspruch 16, bei welchem die Bereiche des genannten seitenumhüllenden Elements (50) gesonderte Seitenumhüllungselement-Bestandteile umfassen.

21. Der absorbierende Artikel (20) nach Anspruch 20, bei welchem die genannten gesonderten Bestandteile des seitenumhüllenden Elements durch eine Naht miteinander verbunden sind.

## Revendications

1. Article absorbant (20) destiné à être porté dans une partie d'entrejambe d'un sous-vêtement, ledit article absorbant (20) ayant une dimension longitudinale s'étendant dans une direction longitudinale (L) et une dimension transversale s'étendant dans une direction transversale (T), ledit article absorbant (20) comprenant:
une partie de corps principal (21) comprenant une âme absorbante (42), ladite partie de corps principal (21) ayant une face tournée vers le corps (20A), une face tournée vers le vêtement (20B) et une paire de bords latéraux longitudinaux (22); et
une paire d'éléments de recouvrement latéraux (50) réunis à ladite partie de corps principal et s'étendant latéralement vers l'extérieur, au-delà des bords latéraux longitudinaux de ladite partie de corps principal vers les bords distaux (54), caractérisé en ce que lesdits éléments de recouvrement latéraux (50) sont prédisposés de manière à s'enrouler autour d'un bord de la partie d'entrejambes du sous-vêtement de l'utilisateur lorsque la partie de corps principal (21) est placée dans une configuration arquée vers le haut, lesdits éléments de recouvrement latéraux (50) comprenant une première partie (5OA) qui est exempte de torons élastiques et qui est principalement extensible dans la direction transversale (T) et une seconde partie moins extensible (50B), disposée latéralement vers l'extérieur par rapport à ladite première partie (50A) qui maintient lesdits éléments de recouvrement latéraux (50) à l'état tendu lorsque ladite première partie (50A) est étendue latéralement vers l'extérieur.

2. Article absorbant (20) selon la revendication 1, dans lequel lesdits éléments de recouvrement latéraux (50) sont capables de prendre (a) une première position stable dans laquelle ils s'étendent généralement vers l'extérieur à partir de ladite partie de corps principal (21); (b) une position instable lorsqu'ils sont partiellement repliés vers la face tournée vers le vêtement (20B) de la partie de corps principal (21); et (c) une seconde position stable dans laquelle lesdits éléments de recouvrement latéraux (50) forment un angle aigu avec la face tournée vers le vêtement (20B) de ladite partie de corps principal (21).

3. Article absorbant (20) selon la revendication 1, dans lequel des sections de ladite partie moins extensible sont également disposées entre des sections de ladite première partie (50A).

4. Article absorbant (20) selon la revendication 3, dans lequel lesdits éléments de recouvrement latéraux (50) comportent un matériau en bande (60) ayant un réseau (62) de zones distinctes susceptible de se déformer qui présente au moins deux degrés de forces de résistance contrôlées en réponse à un allongement axial appliqué le long d'au moins un axe prédéterminé lorsqu'il est soumis à l'allongement axial appliqué dans une direction parallèle audit axe prédéterminé, lesdites zones distinctes comprenant au moins une première zone (64) et une seconde zone (66), ladite première zone (64) ayant une longueur de parcours en surface qui est inférieure à celle de ladite seconde zone (66) lorsqu'elle est mesurée parallèlement audit axe prédéterminé alors que ledit matériau en bande (60) est dans une condition relâchée.

5. Article absorbant (20) selon la revendication 4, dans lequel ladite bande (60) comporte un axe longitudinal (L₁) aligné avec la ligne médiane transversale (T) de l'article absorbant (20) et ledit réseau (62) dudit matériau en bande (60) comporte une partie formée d'un matériau de support, et ladite première zone (64) dudit réseau (62) comporte une pluralité de zones sensiblement planes, linéaires qui sont alignées avec l'axe longitudinal (L₁) dudit matériau en bande (60) et lesdites secondes zones (66) comportent une pluralité de déformations qui sont orientées sensiblement perpendiculairement audit axe longitudinal (L₁) dudit matériau en bande (60).

6. Article absorbant (20) selon la revendication 5, dans lequel certaines parties au moins dudit matériau en bande (60) sont renforcées pour empêcher l'enroulement du bord distal (54) desdits éléments de recouvrement latéraux (50).

7. Article absorbant (20) selon la revendication 6, dans lequel lesdites parties desdits éléments de recouvrement latéraux (50) sont renforcées à l'aide d'une mousseline.

8. Article absorbant (20) selon la revendication 1, dans lequel ladite première partie (50A) comporte une partie souple, adoucie dudit élément de recouvrement latéral (50) et ladite seconde partie (50B) est moins douce et moins souple que ladite première partie (50A).

9. Article absorbant (20) selon la revendication 8, dans lequel ladite seconde partie (50B) est disposée le long du bord distal tout entier (54) dudit élément de recouvrement latéral (50).

10. Article absorbant (20) selon la revendication 8, dans lequel ladite seconde partie (50B) est sensiblement non extensible.

11. Article absorbant (20) selon la revendication 8, dans lequel ladite première partie (50A) comporte une pluralité de zones espacées, adoucies, souples.

12. Article absorbant (20) selon la revendication 8, dans lequel ladite première partie (50A) est extensible et ladite extensibilité est principalement dirigée dans la direction transversale (T).

13. Article absorbant (20) selon la revendication 12, dans lequel lesdites premières parties (50A) ont une dimension mesurée dans la direction longitudinale (L), près du bord distal (54) desdits éléments de recouvrement latéraux (50), et une dimension mesurée au niveau de la partie desdits éléments de recouvrement latéraux (50) près du bord proximal (52) desdits éléments de recouvrement latéraux (50), et ladite dimension mesurée près du bord distal (54) desdits éléments de recouvrement latéraux (50) est plus bande que la dimension adjacente au bord proximal (52) desdits éléments de recouvrement latéraux (50).

14. Article absorbant (20) selon la revendication 13, dans lequel lesdites premières parties (50A) desdits éléments de recouvrement latéraux (50) sont de forme triangulaire.

15. Article absorbant (20) selon la revendication 8, dans lequel lesdites preimières parties (50A) desdits éléments de recouvrement latéraux (50) comportent un matériau choisi dans le groupe constitué des matériaux suivants: un matériau extensible: un matériau mécaniquement modifié: un matériau tendu, un matériau nervuré; un matériau roulé en anneau; un matériau déformé de façon permanente; et un matériau chauffé et étiré.

16. Article absorbant (20) selon la revendication 1, dans lequel au moins l'un desdits éléments de recouvrement latéraux (50) comporte une pluralité de zones qui sont rassemblées longitudinalement vers l'intérieur le long de l'un desdits bords distaux ou proximaux (52, 54) et réunies ensemble dans une condition rassemblée.

17. Article absorbant (20) selon la revendication 16, dans lequel lesdites zones sont rassemblées vers l'intérieur le long du bord distal (54) de l'élément de recouvrement latéral (50).

18. Article absorbant (20) selon la revendication 16, dans lequel lesdites zones sont rassemblées vers l'intérieur le long du bord proximal (52) de l'élément de recouvrement latéral (50).

19. Article absorbant (20) selon la revendication 16, dans lequel les zones dudit élément de recouvrement latéral (50) comportent des sections faisant partie intégrante d'une structure unitaire.

20. Article absorbant (20) selon la revendication 16, dans lequel les zones dudit élément de recouvrement latéral (50) comportent des composants séparés de l'élément de recouvrement latéral.

21. Article absorbant (20) selon la revendication 20, dans lequel lesdits composants séparés dudit élément de recouvrement latéral sont réunis ensemble par un soudage.
